Europäisches Patentamt

European Patent Office

Office européen des brevets

(11) Veröffentlichungsnummer: **0 012 891**
**B1**

(12) **EUROPÄISCHE PATENTSCHRIFT**

(19)

(45) Veröffentlichungstag der Patentschrift:
16.09.81

(21) Anmeldenummer: 79104929.9

(22) Anmeldetag: 05.12.79

(51) Int. Cl.³: **C 07 C 25/08, C 07 C 17/38**

(54) Verfahren zur Herstellung von Dichlorbenzolen.

(30) Priorität: 23.12.78 DE 2855940

(43) Veröffentlichungstag der Anmeldung:
09.07.80 Patentblatt 80/14

(45) Bekanntmachung des Hinweises auf die Patenterteilung:
16.09.81 Patentblatt 81/37

(84) Benannte Vertragsstaaten:
DE FR IT

(56) Entgegenhaltungen:
FR-A-1 374 863

(73) Patentinhaber: BAYER AG, Zentralbereich Patente,
Marken und Lizenzen, D-5090 Leverkusen 1, Bayerwerk
(DE)

(72) Erfinder: Wissner, Adolf, Dr., Am Wasserturm 13,
D-5090 Leverkusen 3 (DE)
Erfinder: Hauser, Werner, Dr., Quellenweg 8,
D-5068 Odenthal (DE)
Erfinder: Bitners, Felix, Dipl.-Ing., Paul-Klee-Strasse 90,
D-5090 Leverkusen 1 (DE)
Erfinder: Wambach, Raimund, Dr.,
Berta-von-Suttner-Strasse 65, D-5090 Leverkusen 1 (DE)

ACTORUM AG.

Verfahren zur Herstellung von Dichlorbenzolen

Die Erfindung betrifft ein Verfahren zur Herstellung von ortho-, meta- und/oder para-Dichlorbenzol durch Auftrennung eines die Dichlorbenzole enthaltenden Isomeren-Gemisches.

Bei der Chlorierung von Benzol oder Monochlorbenzol zu Dichlorbenzol entsteht in der Regel neben Verunreinigungen, die mehr oder weniger als zwei Chloratome am Benzolkern enthalten, ein Gemisch der drei Dichlorbenzolisomeren. Die Verunreinigungen lassen sich im allgemeinen leicht abtrennen, da sie von dem Dichlorbenzol im allgemeinen wesentlich verschiedene physikalische Eigenschaften haben (DE-A 2 332 889).

Dagegen ist die Abtrennung der einzelnen Dichlorbenzole schwierig, da die physikalischen Eigenschaften sehr ähnlich sind.

Besonders schwierig ist die Abtrennung der meta-Verbindung, die praktisch den gleichen Siedepunkt wie die para-Verbindung hat, so dass eine Destillation der DE-A 2 332 889 zufolge unwirksam sein soll. Trennverfahren durch fraktionierte Kristallisation sind kaum durchführbar, da eutektische Gemische entstehen (DE-A 2 332 889).

Zur Lösung dieses Trennproblems ist es bekannt, das Isomerengemisch nach der US-A 3 170 961 zunächst zu bromieren, dann durch Destillation aufzutrennen und darauf wieder zu entbromieren. Dieses Verfahren ist sehr teuer und mit grossen Verlusten bei der Trennung verbunden.

Ein weiteres Trennverfahren mit Hilfe von Molekularsieben (US-A 2 958 708) ist wegen der Schwierigkeiten bei dem Regenerieren der Molekularsiebe und wegen der hohen Kosten ebenfalls unzureichend.

Nach dem Verfahren der DE-A 2 332 889 erfolgt die Trennung des Dichlorbenzol-Isomerengemisches mit Hilfe der Extraktivdestillation in Gegenwart aprotischer Lösungsmittel, deren Dielektrizitätskonstante über 20 und deren Dipolmoment unter 3,0 Debye liegt. Als geeignetes Lösungsmittel wird in der DE-A 2 332 889 Hexamethylphosphorsäuretriamid genannt, das jedoch bei der Anwendung toxikologisch nicht unbedenklich ist (CuEN 17 (1975) und CuEN 3 (1976)). Die anderen in der DE-A 2 332 889 genannten Lösungsmittel haben einen wesentlich niedrigeren Koeffizienten der relativen Flüchtigkeit von meta- und para-Dichlorbenzol. Daraus folgt ein wesentlich höherer Trennaufwand. Ein weiterer Nachteil der Extraktiv-Destillation der Dichlortoluole ist die Notwendigkeit von Spezialkolonnen, die wegen der geringen Grösse der relativen Flüchtigkeit eine grosse Anzahl von Böden bei gleichzeitig geringem Druckverlust pro Boden aufweisen müssen. Diese Kolonnen enthalten, wie in der DE-A 2 332 889 angegeben, geometrisch geformte Maschenfüllkörper aus Metall. Bei der Destillation von chlorierten Verbindungen lässt sich Korrosion, insbesondere bei Maschenfüllkörpern aus Eisenblech oder aus Eisenlegierungen, nicht vermeiden.

Es wurde ein Verfahren zur Herstellung von ortho-, meta- und/oder para-Dichlorbenzolen aus einem Isomerengemisch von Dichlorbenzolen, bei dem man in einem ersten Schritt das Isomerengemisch destilliert, als Sumpfprodukt der Destillationskolonne ortho-Dichlorbenzol abtrennt und über Kopf ein Isomerengemisch erhält, das in einem zweiten Schritt auf eine Temperatur von 10 bis 40 °C gekühlt wird, wobei man als feste Phase para-Dichlorbenzol abtrennt, gefunden, das dadurch gekennzeichnet ist, dass man die dabei erhaltene flüssige Phase in einem dritten Schritt auf eine Temperatur von −40 bis −5 °C kühlt, wobei die hierbei ausfallende feste Phase dem Isomerengemisch für den zweiten Schritt beigemischt wird, und die flüssige Phase in einem vierten Schritt einer weiteren Destillation zuführt, bei der man im Sumpf der Destillationskolonne ein Isomerengemisch erhält, das dem Ausgangsisomerengemisch beigemischt wird, und über Kopf ein Isomerengemisch erhält, das in einem fünften Schritt auf eine Temperatur von −25 bis −40 °C abgekühlt wird und man dabei eine flüssige Phase erhält, die der Destillation des vierten Schrittes zugeführt wird, und reines meta-Dichlorbenzol als feste Phase erhält.

In einer besonderen Ausführungsform des erfindungsgemässen Verfahrens kann man das im vierten Schritt über Kopf der Destillationskolonne erhaltene Isomerengemisch nochmals destillieren. Bei der erneuten Destillation erhält man über Kopf der Destillationskolonne meta-Dichlorbenzol; das Sumpfprodukt wird wieder in die vierte Verfahrensstufe zurückgeführt.

Nach dem erfindungsgemässen Verfahren können Isomerengemische aufgetrennt werden, die ortho-, meta- und para-Dichlorbenzol enthalten. Die Mengen der einzelnen Isomeren im Gemisch können in weiten Grenzen schwanken. Mit Hilfe des Verfahrens können Spuren der Isomeren abgetrennt werden. Es ist jedoch zweckmässig, von Isomerengemischen auszugehen, die mindestens 0,2 Gew.-% eines jeweiligen Isomeren aufweisen. Vorzugsweise können Gemische eingesetzt werden, die mindestens 2 Gew.-% eines jeweiligen Isomeren aufweisen.

Isomerengemische aus Dichlorbenzol erhält man beispielsweise nach bekannten Verfahren durch Chlorierung von Benzol oder Monochlorbenzol (Ullmann, Enzyklopädie der technischen Chemie, Band 9, 4. Auflage, Seite 504 bis 507 (1975) Winnacker-Küchler, Chemische Technologie, Band 3, 2. Auflage (1959) Seite 800/801).

Es ist auch möglich, Dichlorbenzolgemisch durch Dichlorbenzolisomerisierung weitgehend frei von Mono- und Tri- bzw. höherchlorierten Verbindungen zu erhalten.

Zweckmässigerweise trennt man die bei der Herstellung der Dichlorbenzole entstandenen Nebenprodukte, wie Monochlorbenzol oder Tri- und höherchlorierte Benzole vor der Durchführung der erfindungsgemässen Trennung weitgehend ab.

Im allgemeinen erhält man ein weitgehend nur Dichlorbenzol (über 99%) enthaltendes Ausgangsgemisch durch einfache Destillation. Es ist jedoch auch möglich, mit Hilfe der erfindungsgemässen Trennung ein Dichlorbenzol-Isomerengemisch aufzuarbeiten, in dem noch Tri- und höherchlorierte Benzole enthalten sind.

In dem ersten Schritt des erfindungsgemässen Verfahrens trennt man mit einer Destillationskolonne den Hauptteil des ortho-Dichlorbenzols ab. Im allgemeinen wird diese Destillation in einer Kolonne mit 130 bis 250 Böden, vorzugsweise 180 bis 220 Böden durchgeführt, dabei geht über Kopf ein Gemisch aus meta- und para-Dichlorbenzol über, das maximal 2 Gew.-% ortho-Dichlorbenzol enthalten kann. Sind Tri- und höherchlorierte Benzole vorhanden, dann werden diese Nebenprodukte mit dem o-Dichlorbenzol abgetrennt. Die Isolierung des reinen o-Dichlorbenzols erfolgt dann in einer weiteren Destillation.

In dem zweiten Schritt des erfindungsgemässen Verfahrens kühlt man das über Kopf der Destillationskolonne im ersten Schritt des erfindungsgemässen Verfahrens erhaltene Isomerengemisch auf eine Temperatur im Bereich von 10 bis 40 °C ab. Bevorzugt ist der Temperaturbereich von 30 bis 40 °C. Als feste Phase kristallisiert reines p-Dichlorbenzol aus, das abgetrennt wird.

Die in dem zweiten Schritt des erfindungsgemässen Verfahrens flüssige Phase wird in einem dritten Schritt auf eine Temperatur im Bereich von − 5 bis − 40 °C gekühlt. Bevorzugt ist der Temperaturbereich von − 20 bis − 30 °C. Die hierbei ausfallende Phase wird dem Isomerengemisch für die Kristallisation des zweiten Schrittes beigemischt.

Die in dem dritten Schritt des erfindungsgemässen Verfahrens zurückbleibende flüssige Phase wird in einem vierten Schritt einer Destillation zugeführt. Diese Destillation wird in einer Bodenkolonne mit 70 bis 400 Böden, vorzugsweise 200 bis 300 Böden ausgeführt.

Bei der destillativen Trennung können beispielsweise Kolonnen mit mindestens 100 praktischen Böden verwendet werden. Im Sumpf der Destillationskolonne erhält man ein Isomerengemisch, das dem Ausgangsisomerengemisch für den ersten Schritt des erfindungsgemässen Verfahrens beigemischt wird.

Über Kopf der destillativen Trennung im vierten Schritt des erfindungsgemässen Verfahrens erhält man ein Isomerengemisch, das in einem fünften Schritt auf eine Temperatur im Bereich von − 20 bis − 40 °C abgekühlt wird. Bevorzugt ist der Temperaturbereich von − 25 bis − 30 °C.

Als feste Phase fällt hierbei reines m-Dichlorbenzol an. Die flüssige Phase wird der zweiten Destillation des erfindungsgemässen Verfahrens (vierter Schritt) zugemischt. Die Kristallisationsschritte 2, 3 und 5 können selbstverständlich auch in mehrere Einzelschritte aufgeteilt werden.

Die Kristallisationsschritte 2, 3 und 5 des erfindungsgemässen Verfahrens können in handelsüblichen Kristallisationsapparaturen durchgeführt werden, vorzugsweise in Röhrenkristallern, die lösungsmittelfrei arbeiten.

Ebenso können für die Destillationsschritte 1 und 4 des erfindungsgemässen Verfahrens handelsübliche Destillationskolonnen ohne besondere Anforderungen an die Kolonnenkonstruktion eingesetzt werden. Zweckmässigerweise werden Bodenkolonnen verwendet. Die Kolonnen können Glockenbodenkolonnen aus Stahl sein.

Nach dem erfindungsgemässen Verfahren ist es vorteilhafterweise möglich, die Isomeren des Dichlorbenzols quantitativ und ohne Verluste durch das Trennverfahren zu erhalten. Die so erhaltenen Dichlorbenzole sind praktisch frei von anderen Isomeren.

Nachdem in DE-A 23 32 889 angegeben wird, dass einfache Destillation für die Trennung von meta- und para-Dichlorbenzol praktisch unwirksam ist, da die Siedepunkte des meta-Dichlorbenzols und des para-Dichlorbenzols sehr dicht beieinander liegen und auch die Kristallisation nur zu einem eutektischen Gemisch führt, war es überraschend und nicht vorauszusehen, dass nicht nur ein 2-Stoffgemisch (meta-/para-Dichlorbenzol), sondern auch ein 3-Stoffgemisch, nämlich meta-Dichlorbenzol, para-Dichlorbenzol, ortho-Dichlorbenzol sich über eine Kombination von Destillation und Kristallisation so auftrennen lässt, dass man praktisch reines meta-Dichlorbenzol erhält.

Für die destillativen Schritte des erfindungsgemässen Verfahrens können billigere Bodenkolonnen verwendet werden, die weit weniger gegen Korrosion anfällig sind als die im obigen DT-A geforderten Kolonnen mit Maschenfüllkörpern oder geometrisch geformten Drahtnetzen. Es ist bekannt, dass gerade die Chloraromaten durch Anwesenheit geringer Verunreinigungen korrosiv sind, insbesondere dann, wenn geringe Spuren von Wasser anwesend sind. Auch nach einer Trocknung des Dichlorbenzol-Isomerengemisches können geringe Mengen Wasser mit Atmosphärenluft durch Undichtigkeiten in die Destillationskolonne eindringen und Korrosion verursachen. Ein weiterer Vorteil des vorliegenden Verfahrens ist, dass praktisch keine Verluste oder Rückstände bei der Aufarbeitung auftreten.

Das Verfahren ist als umweltfreundlich zu betrachten. Ein weiterer Vorteil ist, dass keine Hilfsstoffe wie Brom oder aprotische polare Lösungsmittel benötigt werden. Ein weiterer Vorteil ist, dass ortho-Dichlorbenzol in der ersten Trennstufe des erfindungsgemässen Verfahrens nicht vollständig abgetrennt werden muss. Durch die Kombination von Kristallisation und Destillation können die Apparaturdimensionen klein gehalten werden.

Dichlorbenzole sind Zwischenprodukte, beispielsweise für Farbstoffe, Riechstoffe, Pharmazeutika, Pflanzenschutz- und Desinfektionsmittel, Lösemittel für Lacke, Öle, Wachse, Harze u.a. und können in der chemischen Industrie als Reaktionsmedien verwendet werden (Ullmanns «Enzyklopädie der techn. Chemie», 4. Auflage, Band 9, Seite 509 (1975), Winnacker-Küchler, Chemische Technologie, Band 3, 2. Auflage (1959), Seite 820).

Beispiel

Ein bei der Chlorierung von Benzol nach der destillativen Abtrennung von Chlorbenzol und niedriger siedenden Verbindungen angefallenes Dichlorbenzol-Gemisch enthält 2,5 Gew.-% m-Dichlorbenzol neben o-Dichlorbenzol 39 Gew.-% und Rest p-Dichlorbenzol, sowie geringe Mengen (unter 5%) Verunreinigungen wie Mono-, Tri-, Tetra-, Penta- und Hexachlorbenzole und auch Spuren anderer Chloraromaten oder auch Chloraliphaten (Dichlorbenzol wird im folgenden auch DCB abgekürzt).

Dieses Gemisch wird in einem ersten Schritt, in einer Bodenkolonne in eine o-Dichlorbenzol-Fraktion, die die höher siedenden Bestandteile enthält und in eine p-Dichlorbenzol-Fraktion getrennt, die die leichter siedenden Bestandteile, also insbesondere m-Dichlorbenzol, enthält. Die m-Dichlorbenzol-Konzentration beträgt ca. 5 Gew.-%. o-Dichlorbenzol wird in einer separaten Destillation, entweder in der gleichen Kolonne oder einer kleineren zweiten Kolonne destillativ von den höher siedenden Verunreinigungen wie Tri-, Tetra-, Penta- und Hexachlorbenzolen getrennt. Aus der ca. 5 Gew.-% m-Dichlorbenzol enthaltenden p-Dichlorbenzol-Fraktion wird nun in einem zweiten Schritt in einem Röhrenkristaller praktisch reines p-Dichlorbenzol (über 99 Gew.-%) isoliert, wobei eine m-Dichlorbenzol enthaltende p-Dichlorbenzol-Fraktion mit einer Konzentration von ca. 30% meta-Dichlorbenzol anfällt.

2. Schritt (Kristallisation bei 30 bis 40 °C)

|  | m-DCB | o-DCB | p-DCB |
|---|---|---|---|
| Einsatzmaterial m-Dichlorbenzol- | 5,1 | 0,3 | 94,6 |
| Konzentrat p-Dichlorbenzol- | 30,0 | 1,8 | 68,2 |
| Kristallisat | Spur | 0,5 | 99,5 |

Die weitere Konzentrierung dieser m-Dichlorbenzol enthaltenden Fraktion wird nun in einem dritten Schritt in einem weiteren Röhrenkristaller durchgeführt.

3. Schritt (Kristallisation bei − 30 °C)

|  | m-DCB | o-DCB | p-DCB |
|---|---|---|---|
| Einsatzmaterial m-Dichlorbenzol- | 30,0 | 1,80 | 68,2 |
| Konzentrat p-Dichlorbenzol- | 77,65 | 3,17 | 19,18 |
| Kristallisat | 18,3 | 1,44 | 80,26 |

Die m-Dichlorbenzol-Konzentrate über 70 Gew.-% werden in einem 4. Schritt, einem Destillationsschritt, zu einem m-Dichlorbenzol-Konzentrat von ca. 95% m-Dichlorbenzol angereichert (das Rücklaufverhältnis ist R/E abgekürzt).

Kolonnen 250 praktische Böden; R/E 80
oder 200 praktische Böden; R/E 150

|  | m-DCB | o-DCB | p-DCB |
|---|---|---|---|
| Einsatzmaterial | 77,5 | 3,17 | 19,18 |
| Produkt aus Kolonnen Kopf | 95,0 | nicht nachweisb. | 5,0 |
| Produkt aus Kolonnen Sumpf | 63,18 | 5,91 | 30,91 |

Die weitere Konzentrierung des m-Dichlorbenzol-Konzentrats mit einer Konzentration von ca. 95% wird in einem 5. Schritt, einer Tieftemperaturkristallisation durchgeführt.

5. Schritt
Kristallisationstemperatur − 30 °C

|  | m-DCB | o-DCB | p-DCB |
|---|---|---|---|
| Einsatzmaterial m-Dichlorbenzol- | 95,0 | unter 0,1 | 5,0 |
| Konzentrat Dichlorbenzol- | 99,5 | − | 0,5 |
| Kristallisat | 90,0 | unter 0,1 | 10,0 |

Dass auch Variationen hinsichtlich der Konzentration wie auch der Verfahrensweise bei den einzelnen Schritten möglich sind, zeigen die nachfolgenden Beispiele:

Varianten bei Schritt 3: (Kristallisation)
Kristallisation bei ca. − 30 °C

|  | m-DCB | o-DCB | p-DCB |
|---|---|---|---|
| Einsatzmaterial m-Dichlorbenzol- | 40,0 | 1,6 | 58,4 |
| Konzentrat p-Dichlorbenzol- | 80,0 | 3,5 | 16,5 |
| Kristallisat | 13,6 | 0,4 | 86,0 |

Kristallisation bei − 30 °C

|  | m-DCB | o-DCB | p-DCB |
|---|---|---|---|
| Einsatzmaterial m-Dichlorbenzol- | 47,9 | 6,0 | 46,1 |
| Konzentrat p-Dichlorbenzol- | 75,0 | 6,25 | 18,75 |
| Kristallisat | 29,8 | 5,8 | 64,4 |

Ausgehend vom 30%igen m-Dichlorbenzol (Schritt 3) kann die Kristallisation in 2 Schritten zum 75%igen m-Dichlorbenzol geführt werden, wobei intermediär eine Fraktion ausfällt, deren m-Dichlorbenzol-Konzentration bei 40–50% liegt.

Varianten beim Schritt 4 (Destillation)
Kolonnen mit 200 praktischen Böden; R/E=180

|  | m-DCB | o-DCB | p-DCB |
|---|---|---|---|
| Einsatzmaterial | 75,0 | – | 25,0 |
| Produkt aus Kol.-Kopf | 95,06 | – | 4,94 |
| Produkt aus Kol.-Sumpf | 54,92 | – | 45,08 |

Kolonnen mit 200 praktischen Böden; R/E=200

|  | m-DCB | o-DCB | p-DCB |
|---|---|---|---|
| Einsatzmaterial | 70,1 | 4,9 | 25,0 |
| Kolonnen Kopf | 96,0 | nicht nachweisb. | 4,0 |
| Kolonnen Sumpf | 55,1 | 7,8 | 37,1 |

Kolonnen mit 200 praktischen Böden; R/E=180

|  | m-DCB | o-DCB | p-DCB |
|---|---|---|---|
| Einsatzmaterial | 70,1 | 4,9 | 25,0 |
| Kolonnen Kopf | 95,8 | nicht nachweisb. | 4,2 |
| Kolonnen Sumpf | 54,9 | 7,9 | 37,2 |

Kolonnen mit 400 praktischen Böden; R/E=50

|  | m-DCB | o-DCB | p-DCB |
|---|---|---|---|
| Einsatzmaterial | 70,1 | 4,9 | 25,0 |
| Kolonnen Kopf | 94,3 | nicht nachweisb. | 5,7 |
| Kolonnen Sumpf | 55,4 | 8,0 | 36,6 |

Kolonnen mit 300 praktischen Böden; R/E=60

|  | m-DCB | o-DCB | p-DCB |
|---|---|---|---|
| Einsatzmaterial | 70,1 | 4,9 | 25,0 |
| Kolonnen Kopf | 94,5 | nicht nachweisb. | 5,5 |
| Kolonnen Sumpf | 55,3 | 8,0 | 36,7 |

Varianten zu Schritt 5
(Kristallisation oder Destillation)
Kristallisationstemperatur − 28 °C

|  | m-DCB | o-DCB | p-DCB |
|---|---|---|---|
| Einsatzmaterial m-Dichlorbenzol- | 94,0 | unter 0,1 | 6 |
| Konzentrat | 99,0 | – | 1 |
| Kristallisationsab- lauge | 89,91 | unter 0,1 | 10,09 |

Sämtliche Kristallisationsvorgänge werden in Röhrenkristallern aus Stahl durchgeführt.

Der Schritt 5 kann auch mit Hilfe einer Destillation in einer Kolonne erfolgen, wie nachfolgende Beispiele zeigen:

Kolonnen mit 200 praktischen Böden; R/E=162

|  | m-DCB | o-DCB | p-DCB |
|---|---|---|---|
| Einsatzmaterial | 96,0 | nicht nachweisb. | 4,0 |
| Kolonnen Kopf | 99,1 | – | 0,9 |
| Kolonnen Sumpf | 84,9 | – | 15,1 |

Kolonnen mit 250 praktischen Böden; R/E=78

|  | m-DCB | o-DCB | p-DCB |
|---|---|---|---|
| Einsatzmaterial | 94,65 | nicht nachweisb. | 5,35 |
| Kolonnen Kopf | 99,05 | – | 0,95 |
| Kolonnen Sumpf | 85,0 | nicht nachweisb. | 15,0 |

Kolonnen mit 300 praktischen Böden; R/E=51

|  | m-DCB | o-DCB | p-DCB |
|---|---|---|---|
| Einsatzmaterial | 96,0 | nicht nachweisb. | 4,0 |
| Kolonnen Kopf | 99,05 | – | 0,95 |
| Kolonnen Sumpf | 84,9 | – | 15,1 |

Kolonnen mit 400 praktischen Böden; R/E=41

|  | m-DCB | o-DCB | p-DCB |
|---|---|---|---|
| Einsatzmaterial | 96,0 | nicht nachweisb. | 4,0 |
| Kolonnen Kopf | 99,1 | – | 0,9 |
| Kolonnen Sumpf | 84,9 | – | 15,1 |

## Patentansprüche

1. Verfahren zur Herstellung von ortho-, meta- und/oder para-Dichlorbenzolen aus einem Isomerengemisch von Dichlorbenzolen, bei dem man in einem ersten Schritt das Isomerengemisch destilliert, als Sumpfprodukt der Destillationskolonne ortho-Dichlorbenzol abtrennt und über Kopf ein Isomerengemisch erhält, das in einem zweiten Schritt auf eine Temperatur von 10 bis 40 °C gekühlt wird, wobei man als feste Phase para-Dichlorbenzol abtrennt, dadurch gekennzeichnet, dass man die dabei erhaltene flüssige Phase in einem dritten Schritt auf eine Temperatur von − 40 bis − 5 °C kühlt, wobei die hierbei ausfallende feste Phase dem Isomerengemisch für den zweiten Schritt beigemischt wird, und die flüssige Phase in einem vierten Schritt einer weiteren Destillation zuführt, bei der man im Sumpf der Destillationskolonne ein Isomerengemisch erhält, das dem Ausgangsisomerengemisch beigemischt wird, und über Kopf ein Isomerengemisch erhält, das in einem fünften Schritt auf eine Temperatur von − 25 bis − 40 °C abgekühlt wird und man dabei eine flüssige Phase erhält, die der Destillation des vierten Schrittes zugeführt wird, und reines meta-Dichlorbenzol als feste Phase erhält.

2. Verfahren nach Anspruch 1, dadurch gekennzeichnet, dass man das im vierten Schritt über Kopf der Destillationskolonne erhaltene Isomerengemisch nochmals destilliert und man dabei das Sumpfprodukt der Destillationskolonne der Destillation des vierten Schrittes zuführt und man meta-Dichlorbenzol über Kopf der Destillationskolonne erhält.

3. Verfahren nach den Ansprüchen 1 und 2, dadurch gekennzeichnet, dass man im ersten Schritt eine Destillationskolonne mit 130 bis 250 Böden verwendet.

4. Verfahren nach den Ansprüchen 1 bis 3, dadurch gekennzeichnet, dass man im vierten Schritt eine Destillationskolonne mit 70 bis 300 Böden verwendet.

**Revendications**

1. Procédé de production d'ortho-, méta- et/ou para-dichlorobenzènes à partir d'un mélange d'isomères de dichlorobenzènes, dans lequel on distille, dans une première étape, le mélange d'isomères, on sépare l'orthodichlorobenzène comme produit de queue de la colonne de distillation et on obtient en tête un mélange d'isomères qu'on refroidit dans une seconde étape, à une température de 10 à 40 °C, et on sépare alors comme phase solide le paradichlorobenzène, caractérisé en ce qu'on refroidit la phase liquide ainsi obtenue, dans une troisième étape, à une température de − 40 à − 5 °C, la phase solide qui précipite alors étant ajoutée au mélange d'isomères destiné à la seconde étape, et on soumet la phase liquide dans une quatrième étape à une seconde distillation dans laquelle on obtient dans le carter de la colonne de distillation un mélange d'isomères que l'on ajoute au mélange d'isomères de départ, et on obtient en tête un mélange d'isomères qui est refroidi dans une cinquième étape à une température de − 25 à − 40 °C et on obtient alors une phase liquide que l'on envoie à la distillation de la quatrième étape, et on obtient comme phase solide du métadichlorobenzène pur.

2. Procédé suivant la revendication 1, caractérisé en ce que l'on distille encore le mélange d'isomères obtenu en tête de la colonne de distillation dans la quatrième étape et on soumet alors le produit de queue de la colonne de distillation à la distillation de la quatrième étape et on obtient du métadichlorobenzène en tête de la colonne de distillation.

3. Procédé suivant les revendications 1 et 2, caractérisé en ce qu'on utilise, dans la première étape, une colonne de distillation ayant 130 à 250 plateaux.

4. Procédé suivant les revendications 1 à 3, caractérisé en ce qu'on utilise dans la quatrième étape une colonne de distillation ayant 70 à 300 plateaux.

**Claims**

1. Process for the preparation of ortho-, meta- and/or para-dichlorobenzenes from an isomer mixture of dichlorobenzenes, in which in a first step, the isomer mixture is distilled, ortho-dichlorobenzene is separated off as the bottom product in the distillation column and an isomer mixture is obtained over the top which is cooled to a temperature of 10 to 40 °C in a second step, para-dichlorobenzene being separated off as a solid phase, characterised in that the liquid phase obtained in this second step is cooled to a temperature of − 40 to − 5 °C in a third step, the solid phase which precipitates during this procedure being admixed to the isomer mixture for the second step, and the liquid phase, in a fourth step, being passed to a further distillation in which an isomer mixture which is admixed to the starting isomer mixture is obtained at the bottom of the distillation column and an isomer mixture is obtained over the top which is cooled to a temperature of − 25 to − 40 °C in a fifth step, and in this procedure a liquid phase which is passed to the distillation of the fourth step is obtained, and pure meta-dichlorobenzene is obtained as a solid phase.

2. Process according to Claim 1, characterised in that the isomer mixture obtained over the top of the distillation column in the fourth step is distilled again, and in this procedure the bottom product from the distillation column is passed to the distillation in the fourth step and meta-dichlorobenzene is obtained over the top of the distillation column.

3. Process according to Claims 1 and 2, characterised in that a distillation column with 130 to 250 trays is used in the first step.

4. Process according to Claims 1 to 3, characterised in that a distillation column with 70 to 300 trays is used in the fourth step.